(19) Europäisches Patentamt
European Patent Office
Office européen des brevets

(11) Numéro de publication : 0 351 312 B1

(12) FASCICULE DE BREVET EUROPEEN

(45) Date de publication du fascicule du brevet : 30.10.91 Bulletin 91/44

(51) Int. Cl.[5] : B01J 29/28, C07C 2/00, C07C 15/00

(21) Numéro de dépôt : 89401994.2

(22) Date de dépôt : 11.07.89

# (54) Catalyseur du type aluminosilicate contenant du gallium et son utilisation en aromatisation des gaz légers C2-C4.

(30) Priorité : 12.07.88 FR 8809632

(43) Date de publication de la demande : 17.01.90 Bulletin 90/03

(45) Mention de la délivrance du brevet : 30.10.91 Bulletin 91/44

(84) Etats contractants désignés : BE DE GB IT NL

(56) Documents cités :
EP-A- 0 119 027
EP-A- 0 215 579
EP-A- 0 303 527

(73) Titulaire : INSTITUT FRANCAIS DU PETROLE
4, Avenue de Bois-Préau
F-92502 Rueil-Malmaison (FR)

(72) Inventeur : Petit, Laurent
10 Rue Alibert
F-75010 Paris (FR)
Inventeur : Bournonville, Jean-Paul
43, Rue des Groues Vauréal
F-95000 Cergy Pontoise (FR)
Inventeur : Guth, Jean-Louis
59, Rue Bellevue Brunstatt
F-68200 Mulhouse (FR)
Inventeur : Raatz, Francis
10, Allée Jacques Prévert
F-78260 Achères (FR)
Inventeur : Kessler, Henri
17, Rue de la Forêt
F-68270 Wittenheim (FR)

EP 0 351 312 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne :

— un catalyseur de type aluminosilicate comprenant une zéolithe de structure MFI, synthétisée en milieu fluorure, contenant du silicium, de l'aluminium, et du gallium et,

— l'utilisation de ce catalyseur dans les réactions d'aromatisation des coupes de gaz légers C2-C4 atomes de carbone par molécule en présence ou non d'oléfines.

Une demande parallèle EP-A-89401993.4 a été déposée le même jour sur des catalyseurs similaires.

La synthèse dans des milieux fluorures de ce type de zéolithe de structure MFI a déjà été décrite dans le brevet français n° 2567868 et plus récemment dans un article de J L. GUTH et coll. (Proc. 7th Int. Zeolite Conf, Tokyo, August 1986, p. 121).

Cette synthèse consiste :

a) dans une première étape à former un milieu réactionnel comprenant de l'eau, une source de silice, une source d'alumine, une source d'un agent structurant pouvant fournir des cations organiques choisis dans le groupe constitué par les cations tétrapropylammonium ($TPA^+$) et tétrapropylphosphonium ($TPP^+$), ce milieu réactionnel contenant en outre des anions fluorures. Le pH du milieu est généralement inférieur à 10 et les rapports molaires des divers constituants du milieu réactionnel sont décrits dans le brevet français n° 2567868,

b) dans une deuxième étape à chauffer le dit milieu réactionnel formé dans l'étape (a) à une température comprise entre environ 80 et 230°C et de préférence entre 140°C et 210°C, cette seconde étape conduisant à l'obtention d'un solide cristallisé, qui est séparé,

c) dans une troisième étape, à chauffer le solide obtenu à l'issue de l'étape b, à une température supérieure à 400°C de manière à éliminer, par décomposition et éventuellement par combustion si le traitement est réalisé en présence d'oxygène, les espèces organiques fournies par l'agent structurant, et contenues dans le solide après synthèse.

Le pH inférieur à 10 du milieu réactionnel peut être obtenu soit directement à partir de l'un ou plusieurs des produits composant le milieu réactionnel, soit par l'ajout audit milieu d'un acide, d'une base, d'un sel acide, d'un sel basique ou d'un mélange tampon complémentaire.

Les anions fluorures $F^-$ peuvent être introduits dans le milieu réactionnel sous forme de fluorures, comme le fluorure de sodium NaF, le fluorure d'ammonium $NH_4F$, le fluorure acide d'ammonium $NH_4HF_2$, le fluorure de tétrapropylammonium $(C_3H_7)_4NF$, le fluorure de tétrapropylphosphonium $(C_3H_7)_4PF$, ou de composés hydrolysables pouvant libérer des anions fluorures dans l'eau, comme le fluorure de silicium $SiF_4$ où le fluorosilicate de sodium $Na_2SiF_6$.

Le fluorure d'ammonium ou le fluorure d'ammonium acide sont des sels préférés, car ils permettent l'obtention d'une zéolithe de structure MFI facile à transformer en sa forme protonée sans qu'il y ait lieu de procéder à des réactions d'échange d'ions.

De nombreuses sources de silice peuvent être utilisées dans la formation du milieu réactionnel. On peut citer, par exemple :

— les silices sous forme d'hydrogels, d'aérogels, de suspensions colloïdales,

— les silices résultant de la précipitation de solutions de silicates solubles, ou de l'hydrolyse d'esters siliciques comme l'ester tétraéthylique de l'acide monoorthosilicique $Si(OC_2H_5)_4$, ou de complexes comme le fluorosilicate de sodium $Na_2SiF_6$ ou d'ammonium $(NH_4)_2SiF_6$,

— les silices préparées par des traitements d'extraction et d'activation de composés cristallisés naturels ou synthétiques, comme les silicates d'aluminium, les aluminosilicates, les argiles, etc...

Les silices utilisées peuvent être divisées ou agglomérées.

Parmi les sources d'alumine utilisables on peut citer les sels d'aluminium (sulfate, nitrate, chlorure, fluorure, acétate par exemple), les hydroxydes et oxydes d'aluminium, les aluminates, les esters comme l'ester tripropylique de l'acide monoorthoaluminique $Al(OC_3H_7)_3$.

Au lieu de partir de sources séparées d'alumine et de silice, on peut aussi utiliser des sources où les deux oxydes sont combinés, comme, par exemple, les gels de silice-alumine amorphes, les aluminosilicates cristallisés, parmi lesquels on peut citer les argiles et les zéolithes.

Les sources de silice et d'alumine peuvent être engagées sous forme soluble ou solide, mais également sous forme d'agglomérats comme des extrudés ou des pastilles. Ce dernier conditionnement convient bien aux sources à base de zéolithes brutes ou modifiées déjà agglomérées, qui peuvent ainsi être transformées, selon le nouveau procédé, en zéolithes préformées.

Les sources d'agent structurant pouvant fournir des cations organiques sont de préférence des cations

tétrahydrocarbylammonium, tétrahydrocarbylphosphonium, hydrocarbyl étant avantageusement alkyle et de manière préférée propyle.

Les cations tétrapropylammonium ($TPA^+$) ou tétrapropylphosphonium ($TPP^+$) qui sont les agents structurants préférés sont ajoutés de préférence sous forme de leurs sels, par exemple les bromures, les fluorures, mais ils peuvent également être engendrés in situ à partir de tripropylamine ou de tripropylphosphine et d'un halogénure de propyle.

Les acides ou sels acides, les bases ou sels basiques ajoutés éventuellement en complément pour amener le pH du milieu réactionnel à la valeur désirée peuvent être choisis parmi les acides courants, comme l'acide fluorhydrique HF, l'acide chlorhydrique HCl, l'acide nitrique $HNO_3$, l'acide sulfurique $H_2SO_4$, l'acide acétique $CH_3COOH$, ou les sels acides comme le fluorure acide d'ammonium $NH_4HF_2$, le fluorure acide de potassium $KHF_2$, le sulfate acide de sodium $NaHSO_4$, le sulfate acide de potassium $KHSO_4$, le phosphate acide de sodium $NaH_2PO_4$ et les bases courantes comme l'ammoniaque $NH_4OH$, la soude NaOH, la potasse KOH ou les sels basiques courants comme les carbonates acide $NaHCO_3$ ou neutre $Na_2CO_3$ de sodium, l'acétate de sodium $CH_3COONa$, les sulfures neutres $Na_2S$ ou acide NaHS de sodium ou les mélanges tampons comme acide acétique $CH_3COOH$-acétate de sodium $CH_3COONa$, ammoniaque $NH_4OH$-chlorure d'ammonium $NH_4Cl$.

La morphologie, la taille et la cinétique de formation de cristaux de zéolithes obtenues selon le procédé de l'invention peuvent être modifiées par l'introduction dans le milieu réactionnel de sels complémentaires comme le chlorure de sodium NaCl, le chlorure de potassium KCl, le chlorure d'ammonium $NH_4Cl$, le sulfate de sodium $Na_2SO_4$ et/ou de cristaux (broyés ou non) de composés solides apparentés aux zéolithes préparées par le procédé de l'invention.

TABLEAU I : Caractéristiques du spectre de diffraction X des zéolithes de structure MFI selon l'invention.

| $d_{hkl}$ (Å) ($10^{-10}$ m) | I/Io | $d_{hkl}$ (Å) ($10^{-10}$ m) | I/Io | $d_{hkl}$ (Å) ($10^{-10}$ m) | I/Io |
|---|---|---|---|---|---|
| 11,08-11,26 | FF | 4,06-4,10 | ff | 2,772-2,793 | ff |
| 9,94-10,20 | mf | 3,99-4,05 | f | 2,725-2,749 | ff |
| 9,68- 9,90 | f | 3,83-3,89 | F | 2,677-2,697 | ff |
| 8,98- 9,08 | ff | 3,80-3,86 | m | 2,648-2,670 | ff |
| 8,00- 8,09 | ff | 3,74-3,78 | mf | 2,605-2,619 | ff |
| 7,40- 7,52 | ff | 3,70-3,74 | mf | 2,581-2,597 | ff |
| 7,03- 7,22 | ff | 3,63-3,67 | mf | 2,545-2,557 | ff |
| 6,64- 6,84 | f | 3,58-3,62 | ff | 2,508-2,526 | ff |
| 6,30- 6,42 | f | 3,46-3,50 | ff | 2,479-2,50 | ff |
| 5,95- 6,07 | f | 3,42-3,46 | f | 2,407-2,419 | ff |
| 5,67- 5,79 | f | 3,38-3,42 | ff | 2,393-2,401 | ff |
| 5,54- 5,61 | f | 3,33-3,37 | f | 2,326-2,340 | ff |
| 5,32- 5,42 | ff | 3,29-3,33 | ff | 2,314-2,332 | ff |
| 5,10- 5,23 | ff | 3,23-3,27 | ff | 2,195-2,209 | ff |
| 5,01- 5,08 | f | 3,16-3,20 | ff | 2,104-2,120 | ff |
| 4,95- 5,03 | f | 3,12-3,16 | ff | 2,077-2,095 | ff |
| 4,84- 4,93 | ff | 3,08-3,12 | ff | 2,070-2,084 | ff |
| 4,59- 4,64 | ff | 3,03-3,07 | f | 2,004-2,022 | f |
| 4,44- 4,50 | ff | 2,976-3,020 | f | 1,985-2,005 | f |
| 4,34- 4,40 | f | 2,943-2,962 | f | 1,944-1,964 | ff |
| 4,23- 4,29 | f | 2,855-2,881 | ff | 1,907-1,922 | ff |

FF = très fort ; F = fort ; mF = moyen à fort ; m = moyen
mF = moyen à faible ; f = faible ; ff = très faible.

Les solides obtenus par la procédure de synthèse décrite précédemment sont des zéolithes de structure MFI dont les diagrammes de diffraction des rayons X ont des caractéristiques correspondant aux spécifications du tableau 1. Ces zéolithes de structure MFI ont comme formule chimique approchée après calcination expri-

mée sous forme oxyde :

$$M_{2/n}O,\ Al_2O_3,\ xSiO_2$$

où x peut varier de 12 à 1000 et où M représente le ou les cations de compensation de valence n. Le point important est que ces solides contiennent, après l'étape de synthèse et également après l'étape d'élimination des composés organiques, de l'élément fluor. La teneur en fluor dans la zéolithe déterminée par analyse élémentaire est comprise pour les solides calcinés, c'est-à-dire ceux résultant de l'étape (c) décrite précédemment, entre 0,02 et 1,5% en poids, avantageusement entre 0,1 et 1,0% et de préférence entre 0,2 et 0,8%.

La présence de fluor dans les zéolithes de structure MFI préparées selon l'invention confère à ces solides des propriétés, notamment des propriétés acides et d'échange ionique, tout à fait différentes de celles des zéolithes de structure MFI synthétisées de manière classique, c'est-à-dire en milieu alcalin (US-A-3.702.886 par exemple). Après synthèse et élimination des composés organiques par calcination (étapes a, b, c), les solides selon l'invention sont caractérisés par un spectre de vibration infrarouge qui présente, comme l'illustre la figure pour des teneurs en fluor de 0,8% (courbe 1), de 0,2% (courbe 2) et de 0,05% (courbe 3) des bandes attribuées classiquement aux groupes Si-OH (zone 3730-3750 $cm^{-1}$) et aux groupes Al-OH structuraux (région 3580-3640 $cm^{-1}$) très peu intenses par rapport à celles d'une zéolithe de structure MFI classique, de même rapport Si/Al égal à 22 (courbe 4% F = 0).

L'absence ou la quasi absence de groupes Al-OH structuraux dans les zéolithes selon l'invention est confirmée par les capacités d'échange ionique de ces solides. En effet les capacités d'échange ionique pour des cations tels que par exemple $Na^+$, $K^+$, $Ga^{3+}$, $Pt(NH_3)_4^{2+}$ etc sont très inférieures aux capacités d'échange théoriques totales que l'on peut calculer à partir de la teneur en aluminium de la charpente cristalline.

Ces solides ne présentant pas ou peu d'hydroxyles structuraux et dont la capacité d'échange est très réduite possèdent de manière surprenante des propriétés acides remarquables. Ainsi la thermodésorption d'ammoniac qui permet de rendre compte de l'acidité globale d'un solide (nombre et force des différents types de sites acides) montre que les solides comprenant du fluor incorporé dans la structure sont très acides. Les spectres de thermodésorption d'ammoniac sont comparables à ceux que l'on obtiendrait avec des zéolithes de structure MFI classiques, cependant l'acidité des solides selon l'invention est d'une nature différente.

Sans nous lier à une théorie particulière, on peut proposer par exemple que ces solides ont, à la place d'une partie au moins des sites Al—(O)—Si≡ classiques, des sites du type :

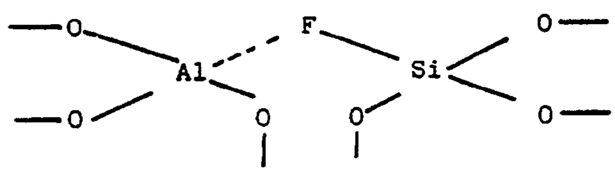

La nature précise des sites acides présents dans les solides selon l'invention reste à préciser, cependant il est clair que ces sites sont liés en grande partie à la présence de fluor et se distinguent par leur nature des sites acides des zéolithes MFI classiques.

L'introduction de fluor dans les zéolithes est une méthode qui a déjà été proposée pour augmenter l'acidité de ces solides (J. MIALE and C. CHANG US 4.540.841). Cependant dans l'art antérieur, le fluor est introduit dans la zéolithe par des modifications réalisées après la synthèse. En d'autres termes, on réalise une synthèse classique, c'est-à-dire en milieu alcalin puis on traite le solide par une technique permettant en principe de fixer du fluor. Ces techniques précédemment proposées, souffrent généralement de gros défauts. Elles sont par exemple, comme c'est le cas quand on traite le solide par du fluor gazeux, susceptibles de conduire à une dégradation de l'ordre cristallin (US-A- 4.297.335). Dans la présente préparation du catalyseur, le fluor est introduit dans la zéolithe au niveau de la synthèse et permet au contraire d'aboutir à des solides très bien cristallisés.

Par des traitements particuliers il est possible d'éliminer partiellement ou totalement le fluor contenu dans les solides entrant dans la composition des catalyseurs selon l'invention sans altérer leur cristallinité. Une technique que l'on peut utiliser pour défluorer les solides consiste à procéder à un traitement dans une solution d'ammoniaque à des températures comprises par exemple entre l'ambiante et 200°C (traitement en autoclave sous pression autogène). L'élimination partielle ou complète du fluor conduit :

— d'une part comme indiqué ci-dessus à l'apparition dans le spectre IR de deux bandes situées vers 3740 et 3608 $cm^{-1}$ correspondant, d'après les attributions admises dans la littérature scientifique, aux groupes silanols terminaux et aux groupes Al-OH structuraux respectivement et

— d'autre part à la restauration de la capacité d'échange ionique telle qu'on peut la calculer à partir de la

teneur en aluminium de charpente des solides.

Ainsi en fonction du traitement de défluoration, on peut obtenir pour un même rapport Si/Al de charpente, des solides contenant une quantité de groupes Al-OH et Si-OH ainsi qu'une capacité d'échange ionique variables. Un solide partiellement défluoré contient donc outre des sites acides classiques du type Al-OH pouvant jouer le rôle de sites d'échanges, des sites acides particuliers dont la nature exacte n'est pas encore totalement élucidée mais qui résultent indéniablement de l'introduction dans les solides de fluor lors de la synthèse.

C'est cette particularité des solides que l'on a mise à profit pour préparer des catalyseurs contenant du gallium et susceptibles par exemple d'aromatiser un hydrocarbure comme le propane et plus généralement une coupe de gaz légers C2-C4 en présence ou non d'oléfines.

La présente invention concerne donc un catalyseur du type aluminosilicate caractérisé par la composition suivante exprimée en poids :

a) 0,01 à 10% en poids de gallium, de préférence 0,03 à 4%,
b) 0,1 à 99,49% d'une matrice choisie dans le groupe formé par l'alumine, la silice, la magnésie, une argile et toute combinaison d'au moins deux des composés précités et
c) 0,50 à 99,99% d'une zéolithe synthétisée en milieu fluorure de formule chimique approchée généralement la suivante :

$M_{2/n}O, Al_2O_3, xSiO_2$, où
M représente un proton et/ou un cation métallique
n est la valence du cation
x est un nombre compris entre 12 et 1000 (rapport molaire $SiO_2/Al_2O_3$)

La zéolithe ayant une teneur en fluor comprise en 0,02 et 1,5% en poids, de préférence 0,1 à 1% en poids, le fluor étant incorporé lors de la synthèse, la dite zéolithe étant aussi caractérisée par un diagramme de diffraction X présenté dans le tableau 1.

Après synthèse en milieu fluorure, le solide peut être soumis si besoins est, à un traitement de défluoration permettant d'ajuster sa capacité d'échange ionique, à la teneur en gallium que l'on veut introduire. Plus la teneur en fluor sera faible, plus la teneur en gallium pourra être élevée.

Le traitement de défluoration est plus ou moins sévère suivant le niveau de défluoration désiré. Il consiste en un ou plusieurs traitements successifs du solide, à reflux dans une solution d'ammoniaque de normalité comprise entre environ 0,05 et 5N et de préférence entre 0,1 et 3N, pendant une durée comprise entre environ 0,5 et 5 heures et de préférence entre 1 et 4 heures avec un rapport V/P défini comme le volume de solution par rapport au poids de solide sec compris entre environ 5 et 50 $cm^3g^{-1}$ et de préférence entre 10 et 30 $cm^3g^{-1}$. Le solide, après chaque lavage est ensuite abondamment lavé à l'eau distillée et séché à l'étuve. Après ces traitements et suivant leur sévérité, la teneur en fluor du solide est comprise entre 0,9 et 0,01% en poids. Si on élimine, par des traitements répétés, sensiblement la totalité du fluor, on aboutit encore à des solides qui se distinguent en particulier par leur spectre IR dans la région 3800-3500 $cm^{-1}$ des zéolithes de structure MFI classiques de même rapport Si/Al de charpente : les solides contenus dans le catalyseur selon l'invention possèdent une proportion plus importante de groupes Si-OH.

Le solide partiellement ou totalement défluoré peut être soumis tel quel au dépôt du gallium ou mis en forme par toutes les techniques connues de l'homme du métier. Il peut en particulier être mélangé à une matrice généralement amorphe, par exemple à une poudre humide de gel d'alumine. Le mélange est ensuite mis en forme, par exemple par extrusion au travers d'une filière. La teneur en zéolithe du support ainsi obtenu est généralement comprise entre environ 0,5 et 99,99% et avantageusement comprise entre environ 40 et 90% poids. Elle est plus particulièrement comprise entre environ 60 et 85% en poids par rapport à l'ensemble zéolithe et matrice.

La teneur en matrice du catalyseur est avantageusement comprise entre environ 10 et 60% et de préférence entre environ 15 et 40% en poids. La mise en forme peut être réalisée avec d'autres matrices que l'alumine, telles que, par exemple la magnésie, la silice alumine, les argiles naturelles (kaolin, bentonite), et par d'autres techniques que l'extrusion telles que le pastillage ou la dragéification. Le gallium est ensuite déposé sur le support par tout procédé connu de l'homme du métier et permettant le dépôt du métal dans la zéolithe. On peut utiliser la technique d'échange cationique avec compétition où l'agent compétiteur est de préférence le nitrate d'ammonium, ou encore la technique de dépôt de gallium sur le catalyseur par imprégnation. Les solutions d'échange ou d'imprégnation du gallium peuvent être préparées à partir de composés du gallium tels que par exemple l'oxyde de gallium, le nitrate de gallium, le sulfate de gallium, des halogénures de gallium ou l'hydroxyde de gallium. Ces techniques d'échange ionique ou d'imprégnation peuvent également être utilisées pour déposer le métal directement sur la poudre de zéolithe, avant son mélange éventuel avec une matrice. La teneur en gallium déposée sur le catalyseur à l'issue de ou des étapes d'échange ionique et/ou d'imprégnation dépend de la teneur en fluor du solide ; elle se situe entre 0,01 et 10% en poids par rapport à l'ensemble du catalyseur et de préférence entre 0,03% et 4,0% en poids.

Le catalyseur de la présente invention, obtenu par les procédures précédentes, est mis en oeuvre pour la réaction d'aromatisation des gaz légers, par exemple du propane et/ou d'un mélange C2-C4 en présence ou non d'oléfines. Cette réaction revêt un intérêt particulier car elle permet de valoriser des résidus d'opérations de raffinage (C2-C4) en des produits à plus haute valeur ajoutée (benzène, toluène, xylènes) tout en contribuant à la production de quantités importantes d'hydrogène indispensable pour les procédés d'hydrotraitement par exemple.

La charge contenant du butane et/ou du propane et/ou de l'éthane, en présence ou non d'oléfines est mise en contact avec le catalyseur de la présente invention à une température comprise entre 400 et 700°C, et plus particulièrement entre 500 et 600°C.

Les exemples qui suivent précisent l'invention sans toutefois en limiter la portée, ils sont données pour une charge constituée uniquement de propane mais sont facilement transposables à une charge plus complexe comprenant un mélange de gaz légers C2-C4 en présence ou non d'oléfines.

Tous les catalyseurs utilisés dans les exemples suivants renferment 20% d'une matrice et 80% de zéolithe.

EXEMPLE 1 : Préparation des zéolithes A et B entrant dans la composition du catalyseur selon l'invention.

On prépare deux zéolithes de structure MFI de rapports atomiques Si/Al proches de 25 et 150 à partir d'une même source d'aluminium et de silicium, à savoir du Tixolex 28 partiellement désaluminé et en utilisant deux rapports atomiques F/Si différents dans les deux mélanges réactionnels.

Le Tixolex 28 est un aluminosilicate de sodium commercialisé par Rhône Poulenc et caractérisé par les rapports atomiques Si/Al = 7,3 et Na/Al = 1,1. On prépare la forme partiellement désaluminée de la manière suivante : 60 g de Tixolex 28 sont agités pendant 3 heures à température ambiante avec 600 ml de $HNO_3$ M/2. Le produit obtenu est filtré et lavé à l'eau jusqu'à pH 7. Après séchage à 80°C, il est conservé dans une humidité relative de 80%. La composition pondérale est la suivante 76,10% $SiO_2$ ; 5,46% $Al_2O_3$ ; 0,24% $Na_2O$ ; 17,63% $H_2O$ totale.

On prépare deux mélanges réactionnels A, B dont les compositions molaires et pondérales sont indiquées dans le tableau 2. Pour cela, on ajoute sous agitation le mélange de $NH_4F$, $N(C_3H_7)_4^+ Br^-$ et eau au Tixolex partiellement désaluminé. La cristallisation des deux mélanges réactionnels A, B est réalisée dans deux autoclaves, dont les revêtements internes sont constitués de polytétrafluoroéthane, à 190°C pendant 3,5 jours.

TABLEAU 2

| | | Tixolex partiellement désaluminé $SiO_2$ | Tixolex partiellement désaluminé $Al_2O_3$ | $NH_4F$ | $N(C_3H_7)_4 Br$ | $H_2O$ |
|---|---|---|---|---|---|---|
| A | moles | 0,2 | 0,0084 | 0,04 | 0,1 | 1,6 |
| | g | 15,8 | | 1,48 | 26,6 | 28,8 |
| B | moles | 0,2 | 0,0084 | 0,25 | 0,1 | 1,6 |
| | g | 15,8 | | 9,25 | 26,6 | 28,8 |

Après cristallisation les solides sont filtrés et lavés avec une solution de diéthylamine à 10% puis avec une solution d'eau chaude. Les solides sont ensuite séchés à 80°C. L'analyse cristallographique montre que les produits A, B sont bien des zéolithes de structure MFI dont le diagramme de diffraction des rayons X correspond aux spécifications du tableau 1. L'analyse chimique des produits A et B après calcination sous air à 550°C est la suivante :

| Produits | A | B |
|---|---|---|
| $SiO_2/Al_2O_3$ molaire | 56 | 280 |
| F (% poids) | 0,8 | 0,5 |

EXEMPLE 2. Catalyseur B1 conforme à l'invention.

Le solide B de l'exemple 1 est mis en forme par extrusion avec un liant ou matrice de type aluminique à raison de 80% en poids de zéolithe et 20% en poids de liant.

Le catalyseur B1 est préparé de la manière suivante : une alumine pseudo boehmite fournie par la société CONDEA est peptisée par ajout d'acide nitrique puis malaxée.

Le catalyseur B1 est obtenu par mélange de cette pseudo boehmite avec la zéolithe B.

Cette zéolithe est introduite à raison de 80 g de zéolithe pour 20 g de liant puis malaxée ; la pâte obtenue, après ajustement de sa consistance par ajout de petites quantités d'eau, est forcée au travers d'une filière de diamètre 1,4 mm, puis séchés sous flux d'air à 120°C et calcinée à 550°C pendant une heure.

Le gallium est déposé sur les extrudés par échange ionique avec compétition. La solution d'échange est préparée à partir de nitrate de gallium $Ga(NO_3)_3$ avec comme agent compétiteur le nitrate d'ammonium $NH_4NO_3$. Le rapport de compétition est égal à environ 10. Le pH de la solution de gallium est ajusté à 2 avec de l'ammoniaque.

Le solide B de départ ayant une teneur en fluor importante (0,5% en poids), la teneur en gallium atteinte après trois échanges successifs est assez faible (0,1% en poids). Le spectre de diffraction X est sensiblement comparable à celui de l'exemple 1. Le catalyseur B1 est testé en aromatisation du propane à 600°C, sous pression atmosphérique. Le propane est dilué par de l'argon dans la proportion voluminique 20% propane pour 80% d'argon. Les performances catalytiques sont reportées dans le tableau 3.

Elles sont définies par :

(conversion du propane % pds) = 100x (masse propane de la charge)-(masse des produits de la recette) /(masse propane de la charge

(sélectivité en B, T, X % poids) = 100x (masse de B+T+X dans la recette)/(masse de propane de la charge)-(masse des produits de la recette)

(rendement en aromatiques % pds) = 100x (masse de B+T+X dans la recette)/(masse de propane de la charge)

EXEMPLE 3 : Catalyseur B2.

Cet exemple montre l'importance du fluor sur les propriétés catalytiques en aromatisation du propane.

On utilise comme zéolithe de départ la zéolithe B de l'exemple 1. La teneur en fluor de 0,5% après décomposition des cations structurants est ramenée à 0% par défluoration en milieu ammoniacal suivant le protocole décrit ci-après :

on fait subit à la zéolithe 3 cycles :

+ solution $NH_4OH$ de concentration 0,2N à 140°C pendant 4 heures

+ filtration et lavage à l'eau distillée

+ séchage à l'étuve à 150°C.

Après traitement, on obtient un solide dont la cristallinité et le rapport Si/Al ne sont pas altérés mais dont la teneur en fluor est d'environ 0%. On introduit alors du gallium (2,45% poids) suivant la procédure décrite dans l'exemple 2 puis le solide est mis en forme avec un liant suivant la description de ce même exemple. Ce catalyseur référencé B2 est testé en aromatisation du propane, les résultats sont reportés dans le tableau 3.

On observe que l'élimination du fluor a permis d'augmenter la capacité d'échange de la zéolithe et donc d'augmenter les quantités de gallium introduites, mais ceci au détriment des propriétés acides et donc des performances catalytiques. Le solide B2 est bien moins actif et sélectif en B, T, X que le catalyseur B1.

<u>EXEMPLE 4</u> : Catalyseur A1 de la présente invention.

Cet exemple montre que la capacité d'échange ionique de la zéolithe peut être ajustée par élimination partielle de fluor tout en conservant les propriétés acides particulières des solides de la présente invention.

On utilise comme zéolithe de départ la zéolithe A de l'exemple 1. Le taux de fluor initial de la zéolithe A après calcinations des cations structurants est ramené à 0,2% par un traitement défluorant en milieu ammoniacal de concentration 0,1 N à 100°C pendant 4 heures. Après traitement le solide est chargé en gallium et mis en forme selon la procédure de l'exemple 2. Le catalyseur référencé A1 qui renferme 0,55% poids de gallium et 0,2% de fluor, avec un rapport $SiO_2/Al_2O_3$ égal à 56, est testé pour la réaction d'aromatisation du propane. On observe à la vue des résultats reportés dans le tableau 3 que l'élimination partielle de fluor a permis d'augmenter la capacité d'échange ionique de la zéolithe et donc d'introduire des quantités plus importantes de gallium, tout en conservant une teneur en fluor suffisante pour préserver les propriétés acides particulière des catalyseurs de la présente invention et donc de bonnes performances catalytiques en aromatisation du propane.

<u>EXEMPLE 5</u> : Catalyseur de comparaison C1 (non conforme à l'invention).

La zéolithe C est une zéolithe de structure MFI synthétisée en milieu basique conventionnel dont la description figure dans le brevet US 3.702.886. Cette zéolithe est synthétisée avec un rapport Si/Al de 240 et ne contient pas de fluor. Après calcination des cations structurants à 550°C suivie de trois échanges en milieu $NH_4NO_3$ 3N, le dépôt de gallium est réalisé conformément à la description de l'exemple 2, c'est-à-dire par échange. La teneur en gallium est équivalente à celle du catalyseur A1. Le solide est mis en forme dans les mêmes conditions que celles décrites dans l'exemple 2 puis est testé en aromatisation du propane. Les résultats sont reportés dans le tableau 3.

On observe qu'en l'absence de fluor, le catalyseur C1 est bien moins actif et sélectif en aromatiques que les catalyseurs de la présente invention.

<u>EXEMPLE 6</u> : Catalyseur de comparaison C2 (non conforme à l'invention).

La zéolithe de départ est la zéolithe conventionnelle C de l'exemple 5. Cette zéolithe subit ensuite une calcination à 550°C suivie de trois échanges en milieu $NH_4NO_3$ 3N. Le solide est ensuite soumis à un traitement à 450°C sous une atmosphère contenant $CH_3F$ pendant 4 heures. La teneur en fluor atteinte à l'issue de ce traitement est de 0,20% en poids. Du gallium est ensuite introduit et le solide mis en forme selon les conditions de l'exemple 2, (c'est-à-dire par échange). Ce catalyseur référencé C2 est testé en aromatisation du propane. Il apparait qu'à teneur en fluor équivalente à celle du catalyseur A1, le catalyseur C2 présente des performances catalytiques médiocres par rapport aux catalyseurs de la présente invention.

TABLEAU 3

| CATALYSEUR | A1* | B1* | B2** | C1*** | C2*** |
|---|---|---|---|---|---|
| Si/Al | 28 | 140 | 140 | 240 | 240 |
| % Ga | 0,55 | 0,15 | 2,45 | 0,55 | 0,36 |
| % F | 0,2 | 0,5 | 0 | 0 | 0,20 |
| PPH | 2,1 | 0,8 | 0,9 | 1,1 | 1,1 |
| Conversion propane | 72,0 % | 41,3 % | 37,0 % | 21,3 % | 5,8 % |
| Rendement B, T, X | 37,4 % | 19,4 % | 15,1 % | 2,6 % | 0,8 % |
| Sélectivité B, T, X | 52,0 % | 47,1 % | 41,0 % | 12,1 % | 14,6 % |

* Zéolithe selon l'invention

** Zéolithe selon l'invention mais défluorée complètement

*** Zéolithe MFI conventionnelle.

## Revendications

1. Catalyseur renfermant en poids :

a) 0,01 à 10% de gallium,

b) 0,1 à 99,49% d'une matrice choisie dans le groupe constitué par l'alumine, la silice, la magnésie, une argile et toute combinaison d'au moins deux des composés précités,

c) 0,50 à 99,99% d'une zéolithe synthétisée en milieu fluorure, de rapport molaire $SiO_2/Al_2O_3$ compris entre 12 et 1000, ladite zéolithe ayant en outre une teneur en fluor comprise entre 0,02 et 1,5% en poids, le fluor étant incorporé lors de la synthèse de la dite zéolithe, laquelle possède un diagramme de diffraction X conforme au tableau 1, ci-dessous :

TABLEAU I : Caractéristiques du spectre de diffraction X des zéolithes de structure MFI selon l'invention.

| $d_{hkl}$ (Å) ($10^{-10}$ m) | I/Io | $d_{hkl}$ (Å) ($10^{-10}$ m) | I/Io | $d_{hkl}$ (Å) ($10^{-10}$ m) | I/Io |
|---|---|---|---|---|---|
| 11,08-11,26 | FF | 4,06-4,10 | ff | 2,772-2,793 | ff |
| 9,94-10,20 | mf | 3,99-4,05 | f | 2,725-2,749 | ff |
| 9,68- 9,90 | f | 3,83-3,89 | F | 2,677-2,697 | ff |
| 8,98- 9,08 | ff | 3,80-3,86 | m | 2,648-2,670 | ff |
| 8,00- 8,09 | ff | 3,74-3,78 | mf | 2,605-2,619 | ff |
| 7,40- 7,52 | ff | 3,70-3,74 | mf | 2,581-2,597 | ff |
| 7,03- 7,22 | ff | 3,63-3,67 | mf | 2,545-2,557 | ff |
| 6,64- 6,84 | f | 3,58-3,62 | ff | 2,508-2,526 | ff |
| 6,30- 6,42 | f | 3,46-3,50 | ff | 2,479-2,50 | ff |
| 5,95- 6,07 | f | 3,42-3,46 | f | 2,407-2,419 | ff |
| 5,67- 5,79 | f | 3,38-3,42 | ff | 2,393-2,401 | ff |
| 5,54- 5,61 | f | 3,33-3,37 | f | 2,326-2,340 | ff |
| 5,32- 5,42 | ff | 3,29-3,33 | ff | 2,314-2,332 | ff |
| 5,10- 5,23 | ff | 3,23-3,27 | ff | 2,195-2,209 | ff |
| 5,01- 5,08 | f | 3,16-3,20 | ff | 2,104-2,120 | ff |
| 4,95- 5,03 | f | 3,12-3,16 | ff | 2,077-2,095 | ff |
| 4,84- 4,93 | ff | 3,08-3,12 | ff | 2,070-2,084 | ff |
| 4,59- 4,64 | ff | 3,03-3,07 | f | 2,004-2,022 | f |
| 4,44- 4,50 | ff | 2,976-3,020 | f | 1,985-2,005 | f |
| 4,34- 4,40 | f | 2,943-2,962 | f | 1,944-1,964 | ff |
| 4,23- 4,29 | f | 2,855-2,881 | ff | 1,907-1,922 | ff |

FF = très fort ; F = fort ; mF = moyen à fort ; m = moyen
mF = moyen à faible ; f = faible ; ff = très faible.

2. Catalyseur selon la revendication 1 renfermant 0,03 à 4% de gallium en poids.

3. Catalyseur selon l'une des revendications 1 et 2 dans lequel la dite zéolithe présente dans son spectre IR deux bandes situées vers 3730-3750 et 3580-3640 $cm^{-1}$ correspondant aux groupes silanols terminaux et aux groupes Al-OH structuraux respectivement.

4. Catalyseur selon l'une des revendications 1 à 3 dans lequel la dite matrice est une alumine.

5. Utilisation du catalyseur selon l'une des revendications 1 à 4 dans les réactions d'aromatisation d'une coupe de gaz légers C2-C4 atomes de carbone par molécule.

## Patentansprüche

1. Katalysator, der in Gew.-% enthält :

(a) 0,01 bis 10% Gallium,
(b) 0,1 bis 99,49% einer Matrix, ausgewählt aus der Gruppe, die besteht aus Aluminiumoxid, Siliciumdioxid, Magnesiumoxid, einem Ton und jeder beliebigen Kombination aus mindestens zwei der genannten Verbindungen,und
(c) 0,50 bis 99,99% eines Zeoliths, der in einem Fluoridmedium synthetisiert worden ist, mit einem Molverhältnis $SiO_2/Al_2O_3$ zwischen 12 und 1000, der außerdem einen Fluorgehalt zwischen 0,02 und 1,5 Gew.-% aufweist, wobei das Fluor bei der Synthese des Zeoliths eingearbeitet worden ist, und der ein Röntgenbeugungsdiagramm entsprechend der folgenden Tabelle 1 aufweist :

Tabelle 1

Charakteristiken des Röntgenbeugungsspektrums der erfindungsgemäßen Zeolithe mit MFI-Struktur

| $d_{hkl}$ (Å) ($10^{-10}$ m) | I/Io | $d_{hkl}$ (Å) ($10^{-10}$ m) | I/Io | $d_{hkl}$ (Å) ($10^{-10}$ m) | I/Io |
|---|---|---|---|---|---|
| 11,08-11,26 | FF | 4,06-4,10 | ff | 2,772-2,793 | ff |
| 9,94-10,20 | mf | 3,99-4,05 | f | 2,725-2,749 | ff |
| 9,68- 9,90 | f | 3,83-3,89 | F | 2,677-2,697 | ff |
| 8,98- 9,08 | ff | 3,80-3,86 | m | 2,648-2,670 | ff |
| 8,00- 8,09 | ff | 3,74-3,78 | mf | 2,605-2,619 | ff |
| 7,40- 7,52 | ff | 3,70-3,74 | mf | 2,581-2,597 | ff |
| 7,03- 7,22 | ff | 3,63-3,67 | mf | 2,545-2,557 | ff |
| 6,64- 6,84 | f | 3,58-3,62 | ff | 2,508-2,526 | ff |
| 6,30- 6,42 | f | 3,46-3,50 | ff | 2,479-2,50 | ff |
| 5,95- 6,07 | f | 3,42-3,46 | f | 2,407-2,419 | ff |
| 5,67- 5,79 | f | 3,38-3,42 | ff | 2,393-2,401 | ff |
| 5,54- 5,61 | f | 3,33-3,37 | f | 2,326-2,340 | ff |
| 5,32- 5,42 | ff | 3,29-3,33 | ff | 2,314-2,332 | ff |
| 5,10- 5,23 | ff | 3,23-3,27 | ff | 2,195-2,209 | ff |
| 5,01- 5,08 | f | 3,16-3,20 | ff | 2,104-2,120 | ff |
| 4,95- 5,03 | f | 3,12-3,16 | ff | 2,077-2,095 | ff |
| 4,84- 4,93 | ff | 3,08-3,12 | ff | 2,070-2,084 | ff |
| 4,59- 4,64 | ff | 3,03-3,07 | f | 2,004-2,022 | f |
| 4,44- 4,50 | ff | 2,976-3,020 | f | 1,985-2,005 | f |
| 4,34- 4,40 | f | 2,943-2,962 | f | 1,944-1,964 | ff |
| 4,23- 4,29 | f | 2,855-2,881 | ff | 1,907-1,922 | ff |

FF = sehr stark; F = stark; mF = mäßig bis stark; m = mäßig;
mF = mäßig bis schwach; f = schwach; ff = sehr schwach

2. Katalysator nach Anspruch 1, der 0,03 bis 4 Gew.-% Gallium enthält.

3. Katalysator nach einem der Ansprüche 1 und 2, in dem der Zeolith in seinem IR-Spektrum zwei Banden aufweist, die bei etwa 3730-3750 und 3580-3640 $cm^{-1}$ angeordnet sind, entsprechend den endständigen Silanolgruppen bzw. den Al-OH-Strukturgruppen.

4. Katalysator nach einem der Ansprüche 1 bis 3, in dem die Matrix ein Aluminiumoxid ist.

5. Verwendung des Katalysators nach einem der Ansprüche 1 bis 4 in Reaktionen zur Aromatisierung einer Fraktion von leichten $C_2$-$C_4$ -Gasen mit 2 bis 4 Kohlenstoffatomen pro Molekül.

## Claims

1. Catalyst containing by weight.

a) 0.01 to 10% of gallium,
b) 0.1 to 99.49% of a matrix chosen from the group constituted of alumina, silica, magnesia, a clay and all combinations of at least two of the compounds mentioned hereinabove.
c) 0.50 to 99.99% of a zeolite synthesized in fluorine medium, with a $SiO_2/Al_2O_3$ molar ratio ranging from 12 to 1000, said zeolite moreover having a fluorine content ranging from 0.02 to 1.5% by weight, the fluorine being incorporated during the synthesis of said zeolite, which has an X-ray diffraction diagram conform with table I, hebelow

TABLE I: Characteristics of the X-ray diffraction spectrum of the zeolites having a MFI structure according to the invention

| $d_{hkl}$ (Å) ($10^{-10}$ m) | I/Io | $d_{hkl}$ (Å) ($10^{-10}$ m) | I/Io | $d_{hkl}$ (Å) ($10^{-10}$ m) | I/Io |
|---|---|---|---|---|---|
| 11.08-11.26 | FF | 4.06-4,10 | ff | 2.772-2.793 | ff |
| 9.94-10.20 | mf | 3.99-4.05 | f | 2.725-2.749 | ff |
| 9.68- 9.90 | f | 3.83-3.89 | F | 2.677-2.697 | ff |
| 8.98- 9.08 | ff | 3,80-3.86 | m | 2.648-2.670 | ff |
| 8.00- 8.09 | ff | 3.74-3.78 | mf | 2.605-2.619 | ff |
| 7.40- 7.52 | ff | 3.70-3.74 | mf | 2,581-2.597 | ff |
| 7.03- 7.22 | ff | 3.63-3.67 | mf | 2.545-2.557 | ff |
| 6.64- 6.84 | f | 3.58-3.62 | ff | 2.508-2.526 | ff |
| 6.30- 6.42 | f | 3.46-3.50 | ff | 2.479-2.50 | ff |
| 5.95- 6.07 | f | 3.42-3.46 | f | 2.407-2.419 | ff |
| 5.67- 5.79 | f | 3.38-3.42 | ff | 2.393-2.401 | ff |
| 5.54- 5.61 | f | 3.33-3.37 | f | 2.326-2.340 | ff |
| 5.32- 5.42 | ff | 3.29-3.33 | ff | 2.314-2.332 | ff |
| 5.10- 5.23 | ff | 3.23-3.27 | ff | 2.195-2.209 | ff |
| 5.01- 5.08 | f | 3.16-3.20 | ff | 2.104-2.120 | ff |
| 4.95- 5.03 | f | 3.12-3.16 | ff | 2.077-2.095 | ff |
| 4.84- 4.93 | ff | 3.08-3.12 | ff | 2.070-2.084 | ff |
| 4.59- 4.64 | ff | 3.03-3.07 | f | 2.004-2.022 | f |
| 4.44- 4.50 | ff | 2,976-3.020 | f | 1.985-2.005 | f |
| 4.34- 4.40 | f | 2.943-2.962 | f | 1.944-1.964 | ff |
| 4.23- 4.29 | f | 2.855-2.881 | ff | 1.907-1.922 | ff |

FF = very strong; F = strong; mF = medium to strong; m = medium;
mf = medium to weak; f = weak; ff = very weak.

2. Catalyst according to claim 1 containing 0.03 to 4%. by weight of gallium.

3. Catalyst according to one of claims 1 and 2 wherein said zeolite shows two bands in its IR spectrum situated around 3730-3750 $cm^{-1}$ and 3580-3640 $cm^{-1}$ corresponding to terminal silanol groups and structural Al-OH groups respectively.

4. Catalyst according to one of claims 1 to 3 wherein said matrix is an alumina.

5. Use of catalyst according to one of claims 1 to 4 in the aromatization reactions of a light-gas cut having $C_2$-$C_4$ carbon atoms per molecule.

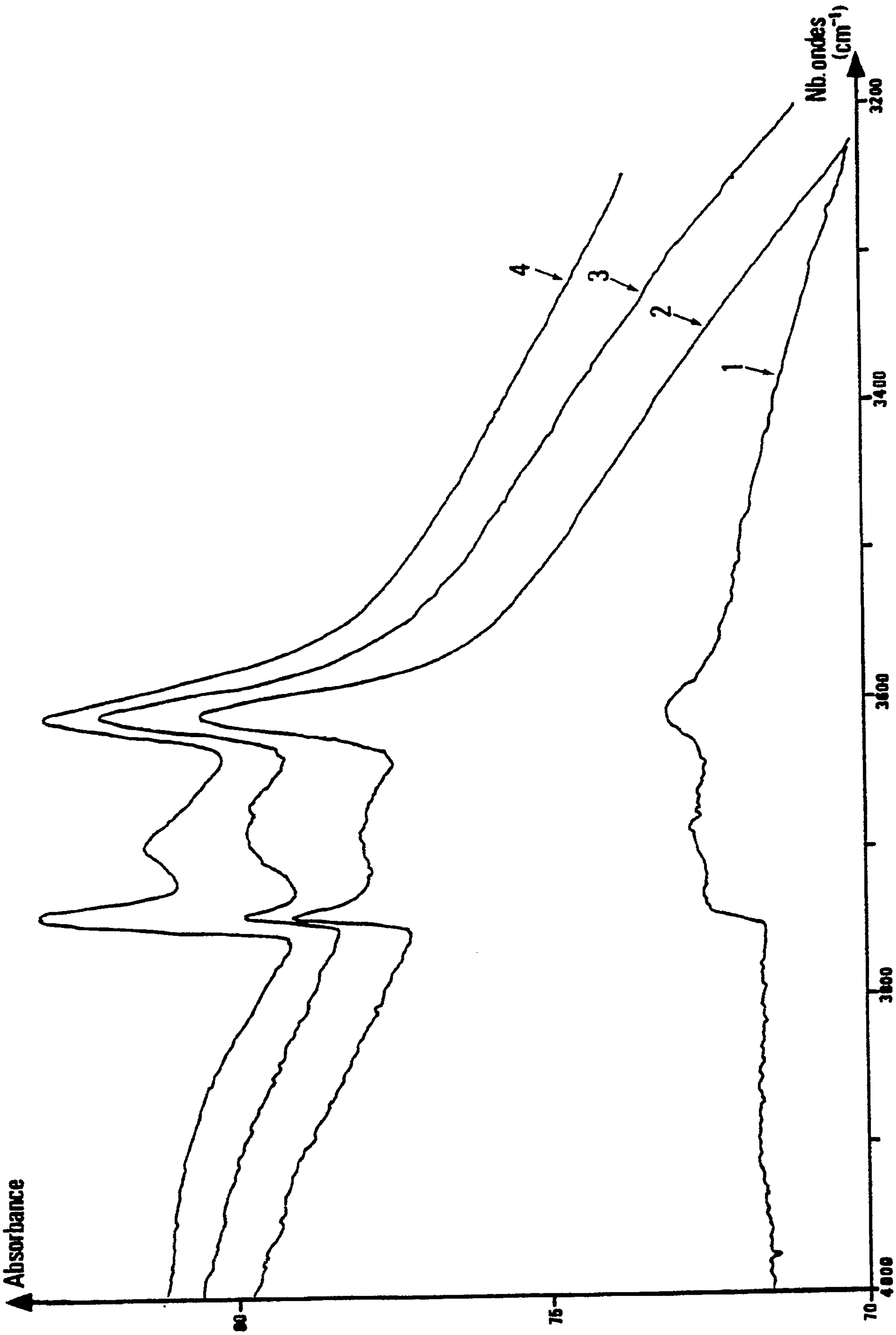
Absorbance
80
75
70
4000
3800
3600
3400
3200
Nb. ondes (cm⁻¹)
1
2
3
4